# EUROPEAN PATENT APPLICATION

(11) **EP 1 295 602 A1**
(43) Date of publication of application: **26.03.2003**
(21) Application number: 01945653.2
(22) Date of filing: 28.06.2001
(51) Int. Cl.: A61K 33/00, A61K 31/02, A61K 31/409, A61K 9/08, A61K 47/02, G02C 7/04, G02C 13/00

(54) **OXYGEN-CONTAINING OPHTHALMIC COMPOSITION**

(30) Priority: 29.06.2000 JP 2000195804
(71) Applicant: ROHTO PHARMACEUTICAL CO., LTD., Osaka-shi, Osaka 544-8666 (JP)
(72) Inventor: NISHIHARA, Toru, c/o Rohto Pharmaceutical Co., Ltd, Osaka-shi, Osaka 544-8666 (JP); SOEJIMA, Yoshiomi, c/o Rohto Pharmaceutical Co Ltd, Osaka-shi, Osaka 544-8666 (JP); KURUMADA, Toshiki, c/o Rohto Pharmaceutical Co Ltd, Osaka-shi, Osaka 544-8666 (JP)
(74) Representative: Stuart, Ian Alexander
(86) International application number: JP0105542
(87) International publication number: WO02000228

(57) **Abstract**

An oxygen-rich ophthalmic composition characterized in that it comprises oxygen and/or oxygen donor, wherein the amount of oxygen and/or oxygen contained in oxygen donor is 10 mg or above per liter of the ophthalmic composition, or the content of oxygen is at least 90% of the saturated dissolved oxygen, which composition is useful in prevention, alleviation, or treatment of ocular diseases, especially corneal diseases or disorders.

## Description

### TECHNICAL FIELD

The present invention relates to an ophthalmic composition enriched with oxygen. In particular, the present invention relates to a composition for preventing or treating ocular diseases caused by hypoxia.

### BACKGROUND ART

The majority of tissues and organs of a living body are supplied oxygen indispensable for their function from circulation blood; however, in the case of cornea, which lacks blood vessels, oxygen is supplied mainly from tear (lacrimal fluid). The oxygen in tear is originated partly from that supplied by blood vessel in conjunctival ring and partly from that dissolved in tear from atmosphere. Accordingly, the oxygen supply from tear is affected by both the external and internal factors. When the cornea and conjunctiva become hypoxic, disorders such as congestion symptom and corneal edema (corneal swelling) first break out, which might lead to, in the long term, the manifestation of various ocular diseases such as keratitis superficialis punctate, corneal vascularization, corneal epithelial erosion, acute corneal epithelial edema, corneal endotherial disorders. Therefore, stable supply of oxygen is essential to maintain the eye, especially cornea normal.

In the naked eye, corneal surface is covered with tear film. The dissolved oxygen in tear can be represented by oxygen partial pressure and is reported as about 155 mmHg when the eyelid is open and about 55 mmHg when the eyelid is closed (Journal of Japan Contact Lens Society 29:23-30, 1987). The cornea uptakes oxygen from the dissolved oxygen in tear in proportion to oxygen partial pressure of tear. When the tear volume decreases, a total amount of dissolved oxygen decreases, and the cornea cannot uptake the required oxygen sufficiently. A deficit in tear volume is caused by, for example, ophthalmic diseases involving lacrimal hyposecretion, decrease of tear secretion due to aging, or the like, decrease of environmental humidity due to air conditioning, or the like, increase of tear evaporation due to long term staring in case of reading, using computer, driving, wearing a contact lens, or the like. Dry eye is generally accompanied by symptoms that are related to, for example, a disease like keratoconjunctival epithelial disorder or xeropthalmia caused by abnormal change of tear in quality or quantity, or a condition causing feelings of desiccation of eyes due to decrease of tear volume. In any cases, decrease or a qualitative change of tear could result in the hindrance to the sufficient protection of eye surface with tear; insufficient oxygen supply to cornea and conjunctiva; the conjunctiva and corneal become liable to disorders and apt to develop conjunctival congestion, corneal edema or corneal swelling.

The dry eye caused by the wearing of contact lens is attributable to the remarkable reduction in tear exchange rate at the time of nictitation, and also to water (moisture) evaporation from the lens surface, etc. To overcome these problems, in the case of a hard contact lens, there have been taken measures such as changing PMMA (polymethylmethacrylate) lens showing non-gas-permeability to a lens showing improved oxygen-permeability (RGP lens, 0₂ lens), or reducing the size of lens so that the tear exchange at the border of the lens can be promoted, and the like. However, there has not been offered yet a contact lens that can prevent the above-mentioned problems completely. On the other hand, in the case of a soft contact lens, which is typically manufactured into a form that fits well to the ocular surface and has a size sufficient to cover the whole cornea. Accordingly, the tear exchange rate at the border of lens is lower than that of a hard contact lens, and oxygen deficiency is liable to occur. In addition, water inside a contact lens easily evaporates from the lens surface, which results in the reduction in tear volume on the corneal surface. Further, it is reported that only a part of water present between a soft contact lens and cornea can move, and hence the lack of oxygen supply to the cornea should be promoted furthermore. Thus, in the case of soft contact lens wearer, a lens contains only a little water inside, and only a small amount of water can move between a lens and the cornea. Accordingly, it requires a considerable time to supplement oxygen that corneal cells consumed, which makes the corneal cells liable to fall into the oxygen deficient conditions.

As mentioned above, a contact lens exerts an influence to corneal oxygen deficiency, and it is highly possible that wearing contact lens, especially for a long term, causes a corneal disorder such as a corneal edema and vascularization due to oxygen deficiency.

In general, eye drops (ophthalmic solutions) or artificial tears are instilled into eyes in order to relieve the dysphoria associated with dry eye or wearing contact lens, or to supplement tears. However, it is difficult for conventional eye drops or artificial tears to supply a sufficient amount of oxygen to cornea for treating or preventing positively the abnormal conditions such as corneal edema, or the like.

### DISCLOSURE OF THE INVENTION

One of purposes of the present invention is to provide an ophthalmic composition which can supply to the cornea and conjunctival abundant oxygen and is useful for preventing or treating ocular diseases or ocular symptoms resulting from oxygen deficiency.

Another purpose of the present invention is to provide an ophthalmic composition which can supply to the cornea a sufficient amount of oxygen and is useful for preventing or treating corneal disorders such as corneal swelling or corneal edema.

Thus, the present invention provides an ophthalmic composition with elevated (increased) oxygen content, i.e., a composition enriched with oxygen. More specifically, the present invention provides the followings:
(1) an ophthalmic composition, which is enriched with oxygen;
(2) the ophthalmic composition set forth in (1), which comprises a sufficient amount of the dissolved oxygen and/or an oxygen donor to treat or prevent an ophthalmological disease resulting from oxygen deficiency;
(3) the ophthalmic composition set forth in (1) or (2), wherein the amount of oxygen present as dissolved oxygen and/or that contained in oxygen donor is 10 mg or above per liter of the composition;
(4) the ophthalmic composition set forth in (1) or (2), wherein the amount of oxygen present as dissolved oxygen and/or that contained in oxygen donor is 8.84 mg/l or above per liter of the composition at 1 atmospheric pressure, 20 °C;
(5) the ophthalmic composition set forth in (1) or (2), wherein the content of oxygen is at least 90% of the saturated dissolved oxygen in the presence of air at 1 atmospheric pressure;
(6) the ophthalmic composition set forth in any one of (1) to (5), which is selected from the group consisting of eye drop, eyewash and contact lens rewetting and/or wetting solution;
(7) the ophthalmic composition set forth in any one of (2) to (6), wherein the oxygen donor is an oxygen carrier comprising captured oxygen;
(8) the ophthalmic composition set forth in (7), wherein the oxygen carrier is fluorocarbon;
(9) the ophthalmic composition set forth in (7), wherein the oxygen carrier is one or more substances selected from the group consisting (a) hemoglobin or a polymer thereof; (b) modified hemoglobin or a polymer thereof, (c) porphyrin complex or a polymer thereof; and (d) a lipid endoplasmic reticulum which includes hemoglobin or a polymer thereof; modified hemoglobin or a polymer thereof; or porphyrin complex or a polymer thereof;
(10) the ophthalmic composition set forth in any one of (1) to (9), which further comprises at least one substance selected from the group consisting of inorganic salts, sugars, vitamins, amino acids, epithelial cell growth factors and oxygen scavengers;
(11) the ophthalmic composition set forth in any one of (1) to (10), which is stored in a container impermeable or less permeable to oxygen;
(12) the ophthalmic composition set forth in any one of (1) to (10), which is stored in an oxygen-permeable container that is encased in a package impermeable or less permeable to oxygen;
(13) the ophthalmic composition set forth in (12), wherein the room formed between the container and the package is replaced with oxygen;
(14) the ophthalmic composition set forth in any one of (11) to (13), wherein the container impermeable or less permeable to oxygen or the package impermeable or less permeable to oxygen comprises at least one substance selected from the group consisting of ethylene-vinyl alcohol copolymer, polyethylene naphthalate and polyethylene terephthalate;
(15) the ophthalmic composition set forth in any one of (11) to (14), which is stored in a unit dosage container;
(16) the ophthalmic composition set forth in any one of (1) to (15), which is useful for preventing or treating keratitis superficialis punctate, corneal vascularization, corneal epithelial erosion, acute corneal epithelial edemas, corneal infiltration or corneal endotherial disorders; and
(17) an oxygen carrier useful for producing the ophthalmic composition set forth in (1).

### BRIEF DESCRIPTION OF DRAWINGS

Fig. 1 is a copy of micrograph of cornea of a rabbit treated with physiological saline. To a rabbit wearing PMMA lens was administered 10 times physiological saline at 30-minute interval, and, six hours later, cornea was removed, stained with hematoxylin eosin (HE staining) and observed under optical microscope.
Fig. 2 is a copy of micrograph of cornea of a rabbit treated in the same manner as described above regarding Fig. 1 except that an ophthalmic composition of the present invention (concentration of dissolved oxygen: 40 mg/L at 20 °C) was administered in stead of physiological saline.

### BEST MODE FOR CARRYING OUT THE INVENTION

With respect to the ophthalmic composition of the present invention, the term "elevated oxygen content" means that the content of oxygen in a composition is positively (artificially) increased. In other words, it means that the ophthalmic composition of the present invention is enriched with oxygen, i.e., contains more (excessive) oxygen than that normally dissolved under the same conditions. The ordinary temperature is between about 15 °C and 25 °C, and the ordinary pressure is around 1 atmospheric pressure (1 atm). Throughout the specification, the composition of the present invention with an elevated oxygen content, i.e., the composition enriched with oxygen, may also be referred to as "oxygen-rich ophthalmic composition" or simply, "oxygen-rich composition".

The oxygen concentration of the ophthalmic composition of the present invention is preferably at least 90%, more preferably equal or above of the saturated dissolved oxygen in the presence of air at 1 atmospheric pressure. The concentration of oxygen in water saturated with dissolved oxygen at 20 °C in the presence of air of 1 atmospheric pressure is about 8.84 mg/L. The concentration of oxygen dissolved in an ordinary ophthalmic composition under the same condition is about 5 mg/L.

The term "oxygen donor" refers to a substance wherein oxygen is captured by a carrier as defined below.

The term "oxygen carrier" refers to a compound having ability (capacity) to capture and transport oxygen. Examples of preferred carrier include those which capture oxygen under high oxygen partial pressure and discharge oxygen under low oxygen partial pressure. Any oxygen donor can serve as an oxygen carrier on condition that said carrier is ophthalmologically acceptable, which includes a compound having ability to transport oxygen, for example, (a) fluorocarbon, (b) hemoglobin or a polymer thereof, (c) modified hemoglobin or a polymer thereof, (d) porphyrin complex or a polymer thereof; and (e) a lipid endoplasmic reticulum, which includes hemoglobin or a polymer thereof, modified hemoglobin or a polymer thereof, or porphyrin complex or a polymer thereof. These oxygen carriers can be used alone or in combination of two or more species.

The term "container impermeable to oxygen" means a container that lacks oxygen-permeability. The term "container less permeable to oxygen" means a container with low oxygen-permeability. Examples of such container include those made from a high gas barrier material, those composed from a high gas barrier material, or those comprising a high gas barrier material. Containers comprising a high gas barrier material can be made from a composite material wherein plastic such as polyethylene and a high gas barrier material are multilayered, subject that such composite does not influence the effect of the present invention.

Examples of "package impermeable to oxygen" and "package less permeable to oxygen" include packages made from or composed of aluminum foil or a high gas barrier material or packages comprising high gas barrier material. The package may be single or multilayered film. Further, the package can be prepared by coating a film made from a material of polyvinyl alcohols or polyamides basis with aluminum, metal oxide such as aluminum oxide, or ceramics such as silicon oxide by vapor deposition method, or with polyvinylidene chloride, or the like. Packages comprising a high gas barrier material can be made from a composite material wherein plastic such as polyethylene and a high gas barrier material are multilayered, subject that such composite does not influence the effect of the present invention.

The term "oxygen-permeable container" refers to a container which has been made from oxygen-permeable material and is free from high gas barrier material, through which oxygen can permeate. Examples of oxygen-permeable material include Teflon, polystyrene, polyethylene or polypropylene etc.

Preferred "high gas barrier material", is a material lacking oxygen-permeability or having low oxygen-permeability. Specifically, oxygen-permeability of such a material is preferably 0 - 2000 ml/m²•day•Mpa, more preferably 0 - 500 ml/m²•day•Mpa, and most preferably 0 - 100 ml/m²•day•MPa, when measurement is conducted on a material of 20 µm thick at 20 °C, 65%RH. Thermoplastics are especially suitable as high gas barrier materials and specific examples include polyamide (nylon 610 etc.), EVOH (ethylene-vinyl alcohol copolymer), PVDC (polyvinylidene chloride), PEN (polyethylene naphthalate), PET (polyethylene terephthalate), and the like. EVOH, PET and PEN are preferred above all. These materials having elevated gas barrier nature can be used after drawing.

For the purposes of the present invention, the following containers or packages are preferable: (a) a container impermeable to oxygen, (b) a container less permeable to oxygen comprising high gas barrier material, (c) a combination of an oxygen-permeable container and a package impermeable to oxygen, or a combination of an oxygen-permeable container and a package less permeable to oxygen. Examples of a container or a package less permeable to oxygen include those made from only a high gas barrier material or a composite material wherein plastic such as polyethylene and high gas barrier material are multilayered, or a resin into which high gas barrier material has been kneaded.

The oxygen-rich ophthalmic composition of the present invention is not limited to any form of as far as it is suited to supply oxygen to cornea; however, it is preferably in the form of aqueous composition, more preferably aqueous solution, suspension or emulsion. Especially preferred form is aqueous solution.

The oxygen content of the present composition containing oxygen and/or oxygen donor is about 8.84 mg or above, preferably about 8.84 - 225 mg, more preferably about 10 - 220 mg, further more preferably about 14 - 180 mg, per liter of the composition. It is generally preferred that a composition contains oxygen at about 10 mg/l or higher concentration as the total of dissolved oxygen and oxygen contained in donor.

The oxygen-rich ophthalmic composition of the present invention preferably contains oxygen at concentration of about 8.84 mg or more per 1 litter. The content of oxygen is more preferably 8.84 - 225 mg, further preferably 10 - 220 mg, and further more preferably 14-180 mg per 1 liter at 1 atmospheric pressure, 20 °C.

The oxygen-rich ophthalmic composition of the present invention preferably contains at least one and a half times oxygen of that contained in an ordinary ophthalmic composition.

### [Processes for Preparing Oxygen-rich Ophthalmic Composition)

The oxygen-rich ophthalmic composition of the present invention can be prepared by any methods used for making a composition containing an excessive amount of oxygen. However, it can be conveniently and preferably prepared by adding oxygen donor, which is consisting of a carrier and oxygen captured, or dissolving excess oxygen gas into a composition. An oxygen-rich ophthalmic composition, which contains oxygen at higher concentration than the saturated dissolved oxygen concentration under ordinary temperature and atmospheric pressure, can be prepared by dissolving oxygen into a composition at low temperature and/or under high oxygen partial pressure. The oxygen-rich ophthalmic composition of the present invention is preferably sterilized in any steps during the formulation process by a known sterilization method such as sterile filtration. Also, it is desirable to conduct the manufacture of oxygen-rich ophthalmic composition under aseptic conditions after the sterilization.

### (1) Production of Ophthalmic Composition Containing Oxygen Donor

Processes of production with various oxygen carriers are illustrated below.

### (A) Process wherein Oxygen Carrier is Fluorocarbon

Fluorocarbons are derived from organic compounds by replacing a part or all of hydrogen atoms with fluorine. In the present method, fluorocarbons are selected from those which are physiologically acceptable and have a capacity to transport oxygen. Fluorocarbon is physiologically acceptable substance because it is excreted from the lung with expiration without undergoing metabolization, even if absorbed into a body.

Examples of fluorocarbon usable in the present invention include perfluorobutyltetrahydrofuran (C₈F₁₆O), which can be represented by the formula (1): 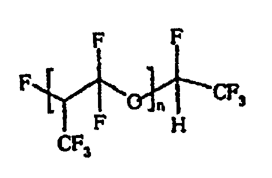 wherein n is 4, perfluorinate ether, perfluorocarbon or perfluoro-tert-amine. They may be used in combination of two or more compounds.

Examples of perfluorocarbon include perfluorocycloalkane, perfluoroalkylcycloalkane, or perfluoroalkane. The carbon number in these compounds is preferably 5 - 20, more preferably 9- 11.

Perfluoroalkylcycloalkane may contain a hetero ring in place of cycloalkane. Examples of perfluoroalkylcycloalkane include perfluoro-C₃₋₅-alkylcyclohexane such as perfluoromethylpropylcyclohexane, perfluorobutylcyclohexane, perfluorotrimethylcyclohexane, perfluoroethylpropylcyclohexane, perfluorodecalin or perfluoromethyldecalin; perfluoro-C₄₋₆-alkyltetrahydropyran such as perfluorohexyl-tetrahydropyran; perfluoro-C₅₋₇-alkyltetrahydrofuran such as perfluoro-pentyltetrahydrofuran, perfluorohexyltetrahydrofuran, or perfluoro-heptyltetrahydrofuran.

Examples of perfluoro-tert-amine include perfluoro-tert-alkylamine, perfluoro-N-C₄₋₆-alkylpiperidine and perfluoro-N-C₅₋₇-alkylmorpholine. The carbon number in these compounds is preferably 3 - 20, more preferably 9-11.

Examples of perfluoro-tert-alkylamine include perfluoro-trialkylamine such as perfluoro-N, N-dibutylmonomethylamine, perfluoro-N, N-diethylpentylamine, perfluoro-N, N-diethylhexylamine, perfluoro-N, N-dipropyi butyl amine, perfluorotripropylamine, or the like; perfluoro-N,N-dialkylcycloalkylamine such as perfluoro-N,N-diethylcyclohexylamine, or the like.

Preferably, a fluorocarbon can be used as an oxygen carrier in the present invention as an emulsion which is stable in physiologically acceptable aqueous medium or is prepared on the occasion of use by suspending the fluorocarbon into an aqueous solvent. For example, an emulsion can be prepared by mixing a fluorocarbon, an emulsifier, optionally an emulsification auxiliary agent, further optionally a solubilizing agent such as salts, alcohols or propylene glycol, a stabilizer, a buffer, water and a pH adjusting agent in a physiologically acceptable aqueous solvent such as distilled water, physiological saline or PBS (phosphate buffered saline) by a method known in the art or a method pursuant thereto. The resultant emulsion is then dispersed into an ophthalmic composition.

The mean diameter of particles in the emulsion is preferably from about 0.05 to about 0.3 µm.

In the process of producing the composition of the present invention, a commercially available fluorocarbons or fluorocarbon mixtures can be used. Examples of commercially available fluorocarbons include inert liquid FC-43, FC-75, FC-77, FC-78, FC-88 (Minnesota Mining Manufacturing Company), and examples of commercially available fluorocarbon emulsion include Fluosol® (Midori-Juji Co., Ltd.)

A liquid fluorocarbon is also usable, which can be prepared according to a method described in, for example, JP H02-271907, A or a method pursuant thereto.

The sterilization of an ophthalmic composition containing fluorocarbon as the oxygen carrier is preferably carried out by a method not affecting the dispersing nature of fluorocarbon emulsion. Furthermore, the fluorocarbon emulsion or liquid fluorocarbon is preferably sterilized before capturing oxygen. Fluorocarbons may condense upon heat sterilization, and, therefore, stabilization is conveniently conducted by a method known to prevent such a problem as shown in JP S48-26912, A.

The step wherein fluorocarbon captures oxygen can be positioned any stage of the total process for preparing the formulation. However, it can be preferably conducted under aseptic conditions after the sterilization. The process for making fluorocarbons capture oxygen can be conducted by known methods, for example, a method comprising dissolving oxygen into fluorocarbon emulsion in an atmosphere of high oxygen partial pressure, a method comprising bubbling oxygen into fluorocarbon emulsion, or a method pursuant thereto.

Because the specific gravity of fluorocarbons is between 1.5 and 2.5, an ophthalmic composition containing fluorocarbon is preferably shaken before use.

### (B) Process wherein Oxygen Carrier is Hemoglobin, Modified Hemoglobin, or Polymer thereof ("Hemoglobin etc.")

Hemoglobin can be obtained, without limitation, by known methods, for example, a method described in JP H11-046759, A, or a method pursuant thereto from a raw material such as erythrocytes, concentrated erythrocyte composition derived from human beings or animals, especially from mammals specifically cow, pig, or the like. Hemoglobin may be a polymer producible in accordance with a known method or a method pursuant thereto.

Examples of modified hemoglobin includes those that can be obtained by binding hemoglobin to polyoxyethylene- or polyethyleneglycol, or the like; treating hemoglobin in the presence of a cross-linking agent such as glutaraldehyde or bis(3,5-bromosalicyl)fumarate as a cross-linking agent. Specific examples of modified hemoglobin include those wherein hemoglobin molecules are linked each other through alpha chains, or hem-binding albumin, and the like. The process for making hemoglobin etc. capture oxygen can be carried out by a known method or a method pursuant thereto.

For example, hemoglobin etc. are made capture oxygen by a process comprising dissolving hemoglobin etc. in a solvent conventionally used in the art, such as physiological saline, PBS or Ringer solution containing lactic acid, and elevating oxygen partial pressure. The oxygen partial pressure should be adjusted depending on the intended amount of oxygen to be dissolved in an ophthalmic composition, but is preferably between about 40 mmHg and about 150 mmHg.

The sterilization of an ophthalmic composition containing hemoglobin etc. as an oxygen carrier can be conducted by a known method.

If hemoglobin undergoes oxidization to give methemoglobin (MetHb) during the process of producing oxygen-capturing hemoglobin or the storage before use, it would lose the capacity to bind to oxygen and results in the deficiency of oxygen transporting ability.

The occurrence of such carriers lacking oxygen-transporting ability can be prevented by a known method. The oxidation rate of hemoglobin depends on temperature, pH, and the like; and hence the oxidation of hemoglobin etc. are avoidable by maintaining the composition at low temperature and/or at pH of about 6.5-9.0. Hemoglobin etc. are preferably prepared and stored at low temperature (about 0-4 °C) and/or at pH ranging from about 6.5 to 9.0.

Furthermore, the oxidation of hemoglobin etc. can be inhibited by adding a reducing agent harmless to human body such as ascorbic acid and the like. Other methods can be used, which are related to enzymatic reduction of hemoglobin etc. (*Biochimicaet Biophysica Acta* **310:**309-316, *Archives of Biochemistry and Biophysics* **77:**478-492) or prevention of oxidization using an alcohol (Biotechnology and Applied Biochemistry 18:25-36).

Also, a method for inhibiting oxidation by attaching an appropriate protective ligand to enzyme binding site of hemoglobin etc. can be used in the present invention. Examples of protective ligand include gaseous ligands such as nitric oxide (NO), non-gaseous competitive ligands such as "derivatives" of carbon monoxide, for example, intros compounds, isocyanide, and the like.

As an oxygen carrier of the present invention, hemoglobin etc. can be encapsulated in a lipid endoplasmic reticulum.

Lipid endoplasmic reticula are morphologically classified into three types, i.e., multilayered liposomes consisting of multiple lipid membranes (Multilamellar Liposome or Vesicle (MLV)), small liposomes consisting of monolayer (Single Compartment Liposome or Small Unilamellar Vesicle (SUV)) and large liposomes consisting of monolayer (Large Unilamellar Vesicle (LUV)). For the present invention, small liposomes (SUNS) are preferred. As a lipid composing an endoplasmic reticulum, any lipid is available as far as it is ophthalmologically acceptable. Examples of such a lipid include phosphatidyl choline (PC), phosphatidyl serine (PS), phosphatidyl ethanolamine (PE), phosphatidyl inositol (PI), lyzophosphatidyl choline (LPC), ganglioside (G), cardiolipin (CL), sphingomyelin (SM), dipalmitoylphosphatidyl choline (DPPC), distearoylphosphatidyl choline (DSPC), phospatidic acid (PA) or phosphatidyl glycerol (PG), or those derived from lipids above through the hydrogenation according to a conventional method.

In addition to a lipid, one or more known substances used in the art may optionally be added, which are selected from the group consisting of, for example, cholesterol (CHOL), dicetyl phosphate (DCP), stearylamine (SA), polyethyleneglycol (PEG), and the like. Cholesterol is also useful in the formation of endoplasmic reticulum, the stabilization of membrane, and the adjustment of permeability of liposome membrane. Further, since dicetylphosphate and stearylamine are charged positively and negatively, respectively, they are useful as a charge donor and thereby adjusting the contact of liposomes and cells. Polyethyleneglycol is also useful in the prevention of agglutination among liposomes.

Furthermore, tocopherol analogs can be added during the formation of endoplasmic reticula . Tocopherol (vitamin E) is a biological element having nonspecific antioxidative effect and can contribute to the stabilization of endoplasmic reticulum membrane or hemoglobin molecules as an antioxidant. In particular, it can be a significant component for the endoplasmic reticulum containing unsaturated lipids.

It is also preferable to add into a lipid endoplasmic reticulum an inhibitor for preventing the conversion of hemoglobin into MetHb. Examples of such inhibitors include sugars involved in the anaerobic glycolytic pathway, a precursor capable of preventing the generation of MetHb. Specific example of a sugar involved in the anaerobic glycolytic pathway is malic acid. Examples of precursors capable of preventing the generation of MetHb include active enzyme scavengers (removers) such as cytochrome b5, NADH-cytochrome b5 reductase, catalase, superoxide dismutase, glutathione peroxidase, or the like.

Also, an allosteric factor of hemoglobin can be included in a lipid endoplasmic reticulum. When hemoglobin etc. are encapsulated in lipid endoplasmic reticula , the former can be present in the aqueous phase of the latter at high concentration, which advantageously results in the reduction of the viscosity as well as colloid osmotic pressure of the resultant ophthalmic composition. However, a lipid endoplasmic reticulum containing purified hemoglobin etc. has much higher affinity to oxygen than erythrocyte and shows extremely high binding activity with oxygen, and hence hardly releases oxygen under low oxygen partial pressure (Circulatory Shock, 31,431 (1990)), which decreases the oxygen transporting capacity (oxygen supplying ability). To avoid such a problem, an allosteric factor of hemoglobin can be included in a lipid endoplasmic reticulum together with hemoglobin etc. This makes it possible to reduce the affinity of hemoglobin etc. for oxygen, which increases the capability of lipid endoplasmic reticula as an oxygen carrier.

Examples of allosteric factor of hemoglobin include 2,3-diphosphoglyceric acid, inosithexaphosphoric acid (IHP), polycarboxylic acid, or condensed phosphoric acid. Further, an electrolyte comprising chloride ion as the negative ion and an organic phosphoric acid derivative are also usable. Examples of electrolyte comprising chloride ion as the negative ion include sodium chloride, potassium chloride, calcium chloride and magnesium chloride. Examples of phosphoric acid derivative include adenosine triphosphate (ATP), adenosine diphosphate (ADP) and pyridoxal-5-phosphoric acid (PLP).

Illustrative examples of allosteric factors are compounds of the formula (2): 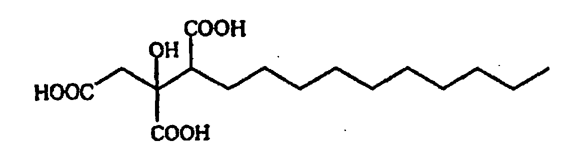 or formula (3): 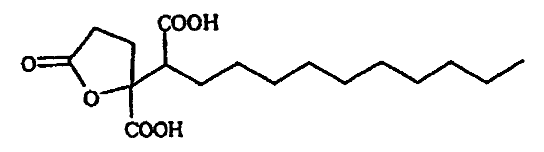 or physiologically acceptable base addition salts thereof. Since the both compounds (2) and (3) have two asymmetric carbon atoms, plural stereoisomers (diastereomer and optic isomers) exist. For the composition of the present invention, there can be used not only a purified isomer but also a mixed compounds comprising any isomers at any proportion, racemate or diastereomer. Compounds of the formulae (1) and (2) can be used alone or in combination at optional ratio. Examples of physiologically acceptable base addition salt include salts with alkaline metal or alkaline earth metal such as sodium salt, potassium salt or calcium salt; salts with organic base such as ammonium salt; methyl amine salt, ethyl amine salt or trimethyl amine salt.

The compounds of the formulae (2) and (3) are known in the art and can be obtained from commercially available ones or produced by a known method or a method pursuant thereto.

The process of preparing a lipid endoplasmic reticulum is known in the art and is not limited to any methods. Lipid endoplasmic reticulum can be prepared in a conventional manner though, there are certain methods preferred, for example, glass beads agitation method, cholic acid elimination method, reversed phase evaporation method, French press method, Ca²⁺ fusion method, peal pumping method, or dehydrate-rehydration method, considering that hemoglobin etc. are liable to undergo oxidation and to change to methemoglobin which lacks oxygen transporting capacity under the influence of temperature, light, hydrogen ion concentration, dissolved gas or metal ions..

Hemoglobin or modified hemoglobin is then made capture oxygen by dissolving the resultant endoplasmic reticula in a solvent conventionally used in the art, such as physiological saline, PBS or lactic acid containing Ringer solution, and increasing the oxygen partial pressure of the solvent. The oxygen partial pressure should be adjusted appropriately depending on the amount of oxygen desired to be dissolved in an ophthalmic composition though, it is generally be preferred to be ranging between about 40 mmHg and about 150 mmHg.

The endoplasmic reticula including hemoglobin etc. as an oxygen carrier of ophthalmic composition of the present invention are preferably stored in a frozen state. To keep stable for a long term in the frozen state, it is preferably to add a freezing protecting agent such as sugar or glycerol. Further, freezing and preservation of endoplasmic reticula can be carried out after adding a compound showing inhibitory effect on agglutination and fusion of endoplasmic reticulum as an stabilizer, which compound is selected from oligo glycolipids, wherein a long chain alkyl is bound to an oligosaccharide through ester binding, and glycoproteins, wherein a long chain alkyl is bound to terminal anomer site of an oligosaccharide chain through ether, amide or ester binding.

### (C) Process wherein Oxygen Carrier is Porphyrin Complex

Porphyrin complexes to be used in the composition of the present invention is derived from ferrous porphyrin (iron (II) porphyrin) complex present in hemoglobin or myoglobin through modification for improving the stability at room temperature, and are shown by the formula (4): 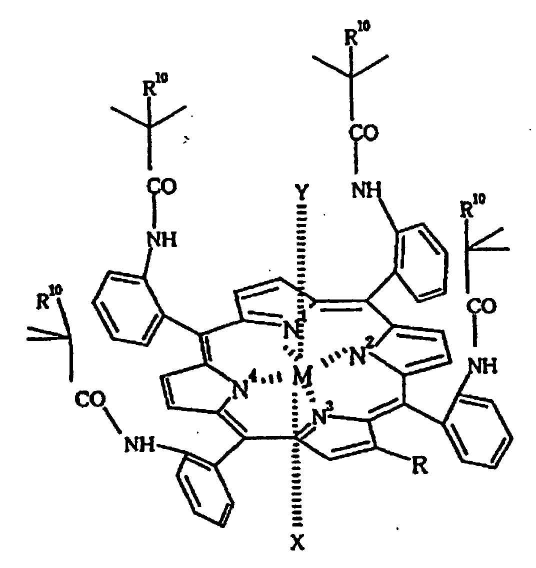 wherein R is hydrogen or a substituent, M is a transition metal ion of fourth or fifth period. The formula (4) above also shows a complex wherein a transition metal ion is absent and a hydrogen atom is bound to each nitrogen atom represented by N² and N⁴. R¹⁰ is a substituent selected from the group consisting of C₁₋₁₈ alkyl and a group of the formula (5): 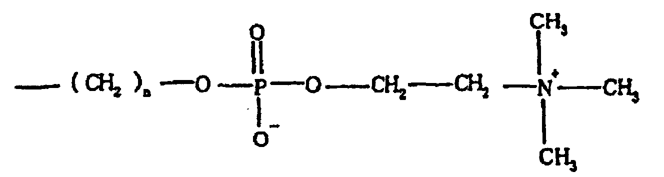 wherein n is the number of methylene group and is selected from the integer of 1-20. X and Y represent a ligand of transition metal ion.

In the formula (4), M is preferably iron(II) or iron(III) ion or cobalt(II) ion.

Among porphyrin complexes of the formula (4), a complex wherein M is iron(II) ion, X and Y each are hydrogen, and R¹⁰ is methyl, i.e., 5,10,15,20-tetrakis(α,α,α,α-o-pivalaimdephenyl)porphyrin iron(II) complex, can bind reversibly to an oxygen molecule(s) at room temperature in the presence of an axis base such as 1-alkyl imidazole or 1-alkyl-2-methyl imidazole in a solvent such as benzene, toluene or an organic solvent such as N,N-dimethylformamide. To apply a porphyrin complex to the present ophthalmic composition, it is preferred to include it into a lipid endoplasmic reticulum as described in (B) above. By doing so, a porphyrin complex of this class can exert the reversible oxygen biding capacity (oxygen-absorption-desorption capacity) even under a physiological conditions (aqueous phase, about pH7.4, about 40 °C or below), and serve as an oxygen carrier for the present invention.

A porphyrin complex of formula (4) wherein R¹⁰ is a substituent shown by the formula (5), which has a structure similar to phospholipid, forms an endoplasmic reticule of bimolecular film when dispersed into water together with phospholipid. In this endoplasmic reticulum, the porphyrin complexes form a layer embedded in such a manner that said complexes are dispersed evenly on the side of a hydrophobic layer, and hence maintains the ability to reversibly bind oxygen (oxygen absorption-desorption capacity) even in an aqueous medium. Accordingly, the said porphyrin complex is preferable as an oxygen carrier of the present invention. Such a porphyrin complex having a structure similar to phospholipid can further be included in a lipid endoplasmic reticulum. When encapsulated in a lipid endoplasmic reticulum, porphyrin complexes can perform the absorption-desorption function repeatedly and stably for a long period of time.

Examples of the substituent R at the 2-position of porphyrin ring of a porphyrin complex of formula (4) include -R'-CHO, -R'-COOH, -R'-COO-R", -COO-R", -R'-NH₂, -R'-SO₃H, C₆₋₂₀ aryl group, hydroxyl group, halogen, formyl group, carbonyl group, amino group, sulfonyl group, azide group, oxy carbonyl chloride or imidazole derivatives, wherein R' and R" represent an aliphatic hydrocarbon chain group. Preferable number of carbon atom of R' is 1-10 while that of R" is 1-20. R and R" may be the same or different from each other. The aliphatic hydrocarbon chain group may be straight or branched, and saturated or unsaturated. Specifically, examples of such group include an alkyl group such as methyl group, ethyl group, n-propyl group, isopropyl group, n-butyl group, isobutyl group, tert-butyl group, pentyl group, isopentyl group, tert-pentyl group, hexyl group, 1,1-dimethylpropyl group, 3-methyl-3-butenyl group, and the like; an alkenyl group such as vinyl group, allyl group, 1-propenyl group, isopropenyl group, 2-butenyl group, 1,3-butadienyl group, 2-pentenyl group, and the like; an alkynyl group such as ethynyl group, 2-propynyl group, 1-butynyl group, 2-butynyl group, or the like. Further, a substituent may have both the double- and triple-bonds as shown by 2-pentene-4-nylyl group. The R" is preferably a straight chain aliphatic hydrocarbon group of carbon number of 1-18. The said chain aliphatic hydrocarbon group may be substituted with halogen. The position and the number of substituent can be selected freely without limitation as far as it is chemically acceptable.

The term "C₆₋₂₀ aryl group" refers to an aromatic hydrocarbon group which may be partially saturated. Examples include phenyl group, benzyl group, biphenyl group, indenyl group, naphthyl group, or a partially saturated derivatives thereof such as 2,3-dihydroindenyl group or 1,2,3,4-tetrahydronaphthyl group, and the like.

Imidazole derivatives as a substituent are shown by the formula (6): 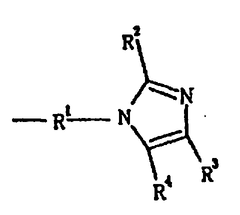 wherein R¹ is C₁₋₂₀ alkylene which is optionally be intervened by -OCONH-, -CONH- or -NHCO- and R², R³ and R⁴ are independently hydrogen or C₁₋₆ alkyl group.

As mentioned above, an imidazole derivative can be added as an axis base into a composition independently from a porphyrin complex. However, the presence of excess amount of an imidazole derivative may result in the expression of its own pharmacological effect or toxicity. Further, in the case where an imidazole derivative is included in a lipid endoplasmic reticulum, it can be one of factors that make the configuration of lipid endoplasmic reticulum unstable. Accordingly, introducing an imidazole derivative into a molecule as a substituent through covalent bond can make the amount of axis base as little as possible.

To facilitate the formation of a covalent bond between a porphyrin complex and a highly fat soluble substance, an appropriate substituent can be introduced at the 2-position of porphyrin ring. For example, a porphyrin complexes bound to a fatty acid through an ester or amide bond can show an improved fat-solubility and hence high solubility in hydrophobic region of a lipid endoplasmic reticulum, which means that such a porphyrin complex can be included into a lipid endoplasmic reticulum easily.

In the case of porphyrin complex of the formula (4) wherein M is a divalent metal, ligands X and Y are generally hydrogen atoms forming coordinate bonds. Replacement of both or either of ligands with an imidazole derivative(s) advantageously reduces the amount of axis base to be added even to the limiting value.

Examples of porphyrin complexes of this form include those wherein an imidazole derivative, as a ligand, is attached to the transition metal M at the center of porphyrin ring through an ester bond (1mole : 1 mole), as shown by the formula (7): 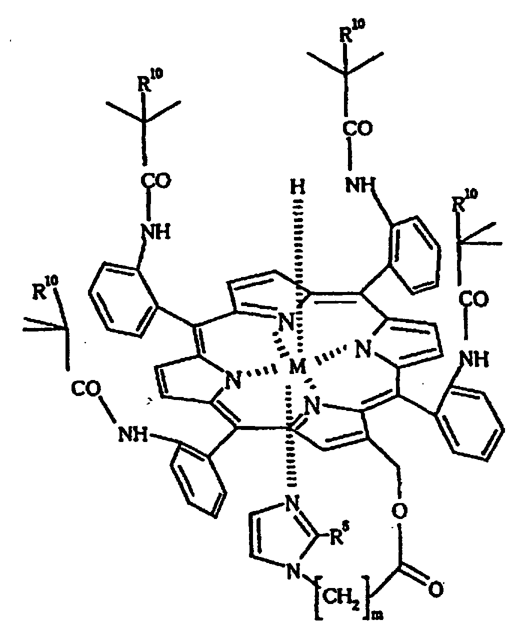 wherein m is an integer of 7-17 and R⁵ is hydrogen or methyl group, and M and R¹⁰ are as defined above. One of advantages of the compound above is that the amount of axis base to be added can be reduced to the limiting value.

A compound having bulky substituents introduced at the both sides of porphyrin ring, which is shown by the formula (8): 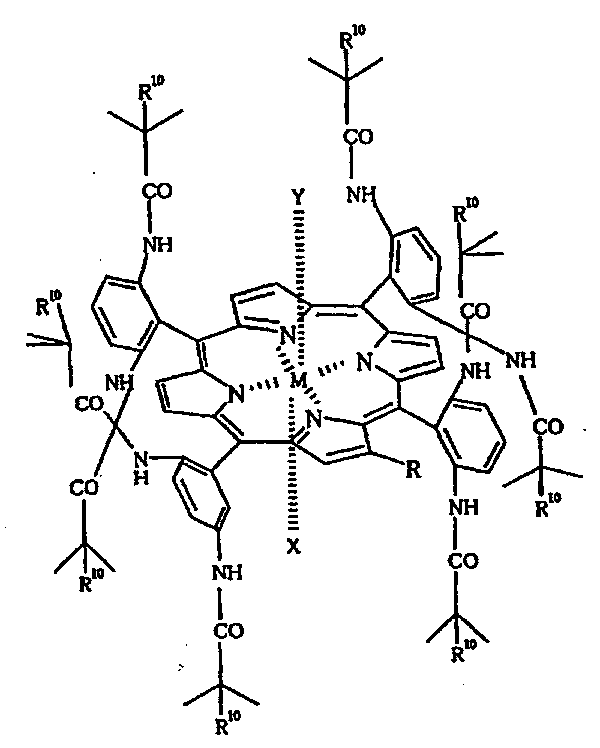 wherein M, X, Y and R¹⁰ are as defined above is also useful. In this case, a porphyrin complex having a bulky substituent at only one side of porphyrin ring is not suited as a carrier for the present invention because such a complex is liable to form µ-dioxo dimmer thereby yielding iron(III) porphyrin complex lacking the oxygen binding activity.

Further, a porphyrin complex of the formula (7) or (8) can be included in a lipid endoplasmic reticulum as mentioned above.

The porphyrin complexes used as an oxygen carrier of the present invention can be produced according to a method such as that described in JP S59-162924, A.

In addition to the porphyrin complex shown by the formula (4), (7) or (8) above, the present invention can use as an oxygen carrier a known synthetic iron(II) porphyrin complexes having oxygen absorption-desorption capacity similar to iron(II) porphyrin complexes present in hemoglobin or myoglobin, as described in J. P. Collman, *Accounts of Chemical Research,* 1977, **10,** 265; Basolo, B. M. Hoffman, J. A. Ibers, *ibid* 1975, **8,** 384.

The oxygen carrier of the present invention can be a polymer of the above-mentioned porphyrin complex. Said polymer can be produced through the polymerization of porphyrin complexes in accordance with a known method or a method pursuant thereto.

To make the porphyrin complexes or polymers thereof above capture oxygen, they are first dissolved in an ophthalmologically acceptable solvent known in the art such as physiological saline, and then oxygen is allowed to go through the solution under high pressure.

The resultant emulsion or solution containing oxygen donor thus produced can be used as an ophthalmic composition of the present invention as it is. However it can be further treated by sterilization, dilution with distilled water or physiological saline, or the like, and/or addition of another substance(s) hereinafter described.

### (2) Production of Ophthalmic Composition without Oxygen Donor

The oxygen-rich ophthalmic composition of the present invention can be produced by a method for increasing the dissolved oxygen without using any oxygen donors. Examples include a method wherein oxygen is incorporated into an ophthalmic composition under oxygen gas atmosphere, specifically, a method where oxygen is bubbled into a composition; a method where oxygen is allowed to dissolve into a composition under high oxygen partial pressure; a method where oxygen is bubbled into a composition under high oxygen partial pressure. In this context, "high pressure" refers to about 1.2 to 5 atmospheric pressure.

Further, dissolved oxygen in water increases in quantity as the temperature goes down and therefore the oxygen-rich ophthalmic composition of the present invention can be prepared under a low temperature condition. The temperature is preferably between about 0 °C - 5 °C. The above-mentioned processes can be conducted under low temperature. Examples include a method where oxygen is bubbled into an ophthalmic composition directly under a condition of low temperature and oxygen atmosphere, or a method where oxygen is allowed to dissolve into a composition under a condition of low temperature and high oxygen pressure. The resultant composition is then optionally subjected to sterilization such as sterile filtration etc. The sterilization treatment can be done under a condition of ambient atmosphere or oxygen atmosphere; however, the latter is preferable because the decrease of dissolved oxygen is inhibited.

Finally, the composition containing excessive oxygen produced according to the process (1) or (2) above is filled into an oxygen-permeable container, encasing the container in a package impermeable or less permeable to oxygen, replacing the room (or space) between the container and the package with oxygen, and sealing up, whereby an oxygen-rich ophthalmic composition which shows almost the same or optionally increased concentration of dissolved oxygen compared to that at the preparation can be obtained.

The oxygen-rich composition of the present invention can contain any ingredients that an ophthalmic composition generally contains subject that they do not adversely affect the oxygen content of the ophthalmic composition. For example, inorganic salts, sugars, vitamins, amino acids, epidermal growth factors, oxygen scavengers, tissue activating agents or metabolite activating agents, corneal epithelium layer disorder therapeutics or corneal epithelium layer expansion enhancing agents, coenzymes or cell-differentiation accelerating agents may be incorporated in the present composition at an ordinary rate used in the art.

Inorganic salts include, for example, potassium chloride, calcium chloride, sodium chloride, sodium bicarbonate, sodium carbonate, magnesium sulfate, disodium hydrogenphosphate, sodium dihydrogenphosphate, potassium dihydrogenphosphate, and the like.

Sugars include, for example, monosaccharides such as glucose etc., disaccharides such as sucrose etc., or polysaccharides such as dextran, cyclodextrin, hyaluronic acid etc., and the like.

Vitamins include, for example, nicotinic acid, nicotinamide-adenine dinucleotide (NAD), flavin adenine dinucleotide (FAD), vitamin Ds (preferably, vitamin D3), vitamin B2, vitamin B6, vitamin B12, panthenol, calcium pantothenate, sodium pantothenate, vitamin A acetate, vitamin E acetate, retinol palmitate, and the like.

Amino acids include, for example, aspartic acid and salts thereof such as magnesium potassium L-aspartate, potassium L-aspartate or magnesium L-aspartate, sodium chondroitin sulfate, and the like.

Epidermal growth factors include, for example, taurine (aminoethylsulfonic acid). Oxygen scavengers include, for example, substances having SOD or SOD-like activity. Tissue activating agents or metabolite activating agents include aspartic acid and salts thereof.

Agents for treating disorder of corneal epithelium layer or enhancing expansion of corneal epithelium layer include polysaccharides such as hyaluronic acid, and biological energy sources such as glucose, and the like.

Coenzymes include, for example, vitamins such as NAD or FAD. Cell-differentiation accelerating agents include, for example, vitamin Ds (preferably vitamin D3), and the like.

Above all, the preferable ingredients for present oxygen-rich ophthalmic compositions include inorganic salts, sugars, vitamins, amino acids, epidermal growth factors and oxygen scavengers. Especially preferable ingredients include glucose, hyaluronic acid, nicotinamide-adenine dinucleotide (NAD), flavin adenine dinucleotide (FAD), vitamin Ds, aspartic acid and salts thereof, taurine, SOD or SOD-like activity substrates, and the like.

Additionally, the present oxygen-rich ophthalmic compositions may contain components ordinary used for ophthalmic compositions in pharmaceutical acceptable amounts. For example, a component(s) selected from the followings may be included, as need arises:
(a) redness relievers such as epinephrine, epinephrine hydrochloride, ephedrine hydrochloride, tetrahydrozoline hydrochloride, naphazoline hydrochloride, naphazoline sulfate, phenylephrine hydrochloride, methylephedrine hydrochloride, and the like;
(b) neostigmine methylsulfate;
(c) anti-inflammatory agents such as glycyrrhizinate dipotassium, epsilon-aminocapronic acid, allantoin, berberine chloride, berberine sulfate, sodium azulenesulfate, zinc sulfate, zinc lactate, lysozyme chloride, and the like;
(d) anti-histamines such as diphenhydramine hydrochloride or chlorpheniramine maleate, and the like;
(e) preservative, for example, quaternary ammonium salt-type preservatives such as benzalkonium chloride or benzethonium chloride; guanidine-type preservatives such as chlorhexidine hydrochloride, chlorhexidine gluconate, dodecylguanidine chloride or 6-acethoxy-2,4-dimethylmetadioxane, chlorobutanol, and the like;
(f) thickeners such as polyvinyl alcohol, polyvinylpyrrolidone, hydroxyethyl cellulose, hydroxypropylmethyl cellulose, hydroxymethyl cellulose, and the like;
(g) buffering agents such as boric acid, and the like;
(h) pH adjusting agents such as borate, and the like;
(i) solubilizing agents such as polyoxyethylene hydrogenated castor oil, and the like;
(j) ethylenediaminetetraacetatic acid (EDTA) or salts thereof; and
(k) refreshing agents such as camphor, menthol, and the like.

It is preferred to adjust the pH of the composition of the present invention at about 4 to 9. However, when the oxygen carrier is hemoglobin etc., it is preferable to maintain the pH between about 6.5 and 9.0 to inhibit oxidation as mentioned above.

The oxygen rich composition of the present invention can maintain a high oxygen content in an airtight container; however, it is desirable to keep the composition in a container impermeable or less permeable to oxygen as defined above so as to prevent the decrease of oxygen content as the time passes and to ensure the stable supply of oxygen to cornea for a long term (generally, about 1 month to 3 years). Further, the container may be encased in a package impermeable or less permeable to oxygen as defined above and sealed. Optionally, an oxygen-rich ophthalmic composition can be filled in an oxygen-permeable container, encased in a package impermeable or less permeable to oxygen as defined above, and then sealed. This makes it more certain that an effective oxygen concentration is maintained.

In any cases, when an oxygen rich ophthalmic composition is filled in a container, it is preferred to replace the void space in the container with oxygen or to make the inside void space rate not higher than 10%.

Further, the preparation and preservation of the present oxygen rich ophthalmic composition can be facilitated by encasing an oxygen-permeable container containing an oxygen-rich ophthalmic composition or an ophthalmic composition prepared in a conventional manner in a package impermeable or less permeable to oxygen, sealing and replacing the room between the said container and package with oxygen gas. Thus, as oxygen permeates from package into oxygen-permeable container, the oxygen partial pressure in the void space of container elevates, which leads to the increase of the dissolved oxygen concentration in the composition. This embodiment is also convenient for preventing the dissolved oxygen in the composition from decreasing.

The amount of oxygen to be filled in the room between an oxygen permeable container and a package impermeable or less permeable to oxygen should be sufficient to maintain the desired equilibrium between the oxygen atmosphere in the void space and the dissolved oxygen in the ophthalmic composition in an airtight environment. It is preferred to replace about 50% or more of the space volume with oxygen gas. Because the equilibrium between the oxygen atmosphere in the room surrounding the container and the dissolved oxygen in the ophthalmic composition is thus achieved, the dissolved oxygen can be maintained for a long term.

A composition of the present invention, when a container is encased in a package, can be prepared according to a process comprising, for example, the following steps:
(1) filling an ophthalmic composition containing oxygen carrier or an ordinary ophthalmic composition into a container;
(2) incorporating oxygen into the composition above;
(3) closing tightly the container above and encasing the container in a package impermeable or less permeable to oxygen;
(4) filling oxygen into the room between the container and the package and sealing.

Alternatively, it can be prepared according to a process comprising the following steps:
(1) incorporating oxygen into an ordinary ophthalmic composition;
(2) filling the oxygen-rich ophthalmic composition prepared in (1) above into a container;
(3) closing tightly the container above and encasing the container in a package impermeable or less permeable to oxygen; and
(4) filling oxygen into the room between the container and the package and sealing.

The sterilization step can be positioned before or after of any one of steps (1) to (4) of processes above; however, preferred position is before the step (2) in case of the former process, and before the step (1) in case of the latter process. The steps following the sterilization are preferably conducted under an aseptic condition. It is also preferable that the processes further comprise a step for replacing the void space in a container with oxygen before closing in step (3).

In the present invention, it is especially preferred to fill the oxygen-rich composition into a container impermeable or less permeable to oxygen.

There is no restriction on the form, size, and the like of container into which the present composition is filled; however, such containers generally have the form of rectangle or cylinder with a capacity ranging from about 0.2mL to 30mL, which are conveniently applied to the present invention.

A blow-fill-seal container is preferred since it is highly airtight. A blow-fill-seal container comprising high gas-barrier material is especially preferred because it can preserve an oxygen-rich ophthalmic composition stably for a long term.

There is no specific restriction on the form, size, and the like of package subject that it can accommodate the container above. In case that oxygen is filled in the room between a package and a container, the package preferably has a shape and size suited to provide a room of sufficient volume between the container encased. In general, a package is preferably has about 1.2 to about 2 times the volume of the-above mentioned plastic container.

When the present composition is encased in a package, it is preferred that a composition of unit dosage form is included in a container that is encased in a package.

In the process for producing an oxygen-rich composition of the present invention, it is preferred that the steps before the step for filling the composition into a container and also the step for filling the composition into a container are conducted under low temperature or an oxygen atmosphere. In case that the sterilization is done beforehand, the filling step is preferably conducted under aseptic conditions.

The ophthalmic composition of the present invention is used as, for example, ophthalmic solutions, eye wash or contact lens-fitting solution, or the like. When using the ophthalmic composition of the present invention as ophthalmic solutions or eyewash, it can be used at any occasion before, after or while using a contact lens including hard or soft contact lens.

The ophthalmic composition of the present invention is useful in prevention or treatment of ocular diseases associated with hypoxia, for example, punctate disorders such as keratitis superficialis punctate; corneal vascularization; plane disorder including disorders of corneal epithelium such as epithelial erosion or acute corneal epithelial edemas; and also other disorders such as vascular infiltration or disorders of corneal endothelium. The present ophthalmic composition is effective for preventing or treating the above-mentioned diseases/disorders irrespective of the causes, however, is suitable in prevention or treatment of ocular diseases associated with dry eye in a subject who is wearing contact lens or using computer. The present composition is also useful for supplementing tear.

The present invention is further described in the following Examples, but should not be construed as limiting the scope of the present invention.

### Example 1: Ophthalmic Solutions

| | |
|---|---|
| Sodium chloride | 0.700g |
| Potassium chloride | 0.100g |
| Boric acid | 1.000g |
| Borate | 0.200g |
| Disodium Edetate | 0.050g |
| Sorbic acid | 0.1g |
| Solution of 0.1 N sodium | p.r.n. |
| hydroxide in water | |
| 0.1 N Hydrochloric acid | p.r.n. |
| Sterile purified water | p.r.n. |
| Total | 100ml |

Disodium edetate was dissolved gradually in 80 ml of water. To the disodium edetate solution were added boric acid and borate. After sodium chloride and potassium chloride were dissolved, sorbic acid was added and dissolved in the solution. The resultant solution was adjusted to about pH 7.4 using 0.1 N sodium hydroxide and 0.1 N hydrochloric acid solutions, and the total volume was adjusted to 100 ml. Oxygen gas was bubbled in the solution under oxygen gas atmosphere at 1 atm, 20°C for 30 minutes to supersaturate the dissolved oxygen. Then, under oxygen gas atmosphere, the solution was filtered through cellulose acetate filter (0.2 µm pore-size), filled into sterile plastic containers of polyethylene naphthalate and the void space was replaced with oxygen, and the container was closed with a polypropylene cap, encased in a package of a composite film mainly consisting of polyvinyliden chloride, and the room between the container and the package was replaced with oxygen. The ophthalmic solutions of the present invention was obtained after heat-seal. The dissolved oxygen concentration at 20°C was 40 mg/L, which was about 4.5-fold of saturated dissolved oxygen concentration.

### Example 2: Ophthalmic Solutions

A solution was prepared from the same components according to the same method as described in Example 1. Oxygen gas was bubbled in the solution under oxygen gas atmosphere at 1 atm, 20°C for 30 minutes to supersaturate the dissolved oxygen. Under air atmosphere, the solution was then filtered through cellulose acetate filter (0.2 µm pore-size) and filled into sterile plastic containers of polyethylene naphthalate, void space was replaced with oxygen and the container was closed with an aluminum screw cap furnished with a polypropylene packing to provide the ophthalmic solutions of the present invention. The dissolved oxygen concentration at 20°C was 9 mg/L.

### Example 3: Ophthalmic Solutions

A solution was prepared from the same components according to the same method as described in Example 1. Oxygen gas was bubbled in the solution under oxygen gas atmosphere at 1 atm, 5°C for 30 minutes to supersaturate the dissolved oxygen. The solution was filtered through cellulose acetate filter (0.2 µm pore-size) and filled into sterile plastic containers of polyethylene terephthalate and the container was heat-sealed to provide the ophthalmic solutions of the present invention. The dissolved oxygen concentration at 5°C was 10 mg/L.

### Example 4: Ophthalmic Solutions

A solution was prepared from the same components according to the same method as described in Example 1. Oxygen gas was bubbled in the solution under oxygen gas atmosphere at 1 atm, 5°C for 30 minutes to supersaturate the dissolved oxygen. Under oxygen gas atmosphere at 5°C, the solution was filtered through cellulose acetate filter (0.2 µm pore-size) and filled into sterile plastic containers (EVOH) and then worked up as described in Example 1 to provide the ophthalmic solutions of the present invention. The dissolved oxygen concentration at 20°C was 180 mg/L, which was about 20-fold of saturated dissolved oxygen concentration.

### Example 5: Ophthalmic Solutions

To 60 ml of distilled water were added 2.5 g of glycerin and 1.5 g of polyoxyethylene hydrogenated castor oil (Nikko chemicals Inc.), and the mixture was heated at 65°C to dissolve the components.

To the solution was added gradually 20 g of fluorocarbon (Inert Liquid FC-75, Minnesota Mining & Manufacturing Co.) pre-warmed at 60 °C by injection with vigorous stirring to provide a crude emulsion.

Separately, 0.05 g of sodium edetate was dissolved gradually in 20 ml of water. To the sodium edetate solution were dissolved 1.0 g of boric acid and 0.2 g of borate, and 0.7 g of sodium chloride and 0.1 g of potassium chloride were dissolved. After 0.1 g of sorbic acid was added and dissolved, the pH of the solution was adjusted to about 7.4 with 0.1 N sodium hydroxide and 0.1 N hydrochloric acid.

This solution was added to the above crude emulsion and pH was adjusted to about 7.4 with 0.1 N sodium hydroxide and 0.1 N hydrochloric acid. The total volume was then adjusted to 100 ml by adding distilled water. This solution was circulated with a high pressure emulsifier (Manton-Gaulin Emulsifier) under pressure at 450 atm for 5 minutes to provide an emulsion. Oxygen gas was bubbled in the solution at 1 atm, 20°C for 30 minutes to make the oxygen-carrier, Inert Liquid FC-75 (Minnesota Mining & Manufacturing Co.) capture oxygen. The amount of captured oxygen was 15 mg/L. Under oxygen gas atmosphere, the solution was then filtered through cellulose acetate filter (0.2 µm pore-size) and filled into a sterile plastic container of polyethylene naphthalate, which container was encased in a package of composite film mainly consisting of polyvinyliden chloride and the room between the container and the package was replaced with oxygen. The ophthalmic solutions of present invention was obtained after heat-seal.

### Test Example 1: Corneal Edema Inhibition Test

A contact lens of PMMA (polymethylmethacrylate) adapted to a rabbit cornea was applied to a white house rabbit. The ophthalmic solutions prepared in Examples 1-3 were instilled into eye 10 times every 30 minutes and 5 hours later, the corneas were removed and excised to prepare sections. The sections were stained with HE staining (hematoxylin eosin staining) and observed under optical microscope.

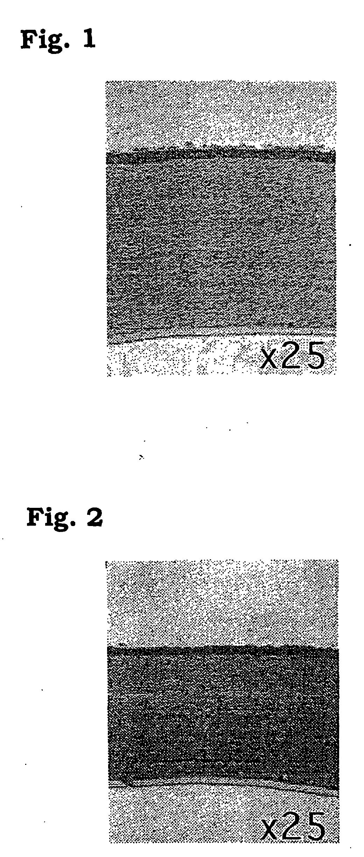

As control, saline was instilled into eye 10 times every 30 minutes for comparison. The corneas were stained with HE staining as described above and observed under optical microscope. The results are shown in Figs. 1 and 2. As shown in Fig. 1, when saline was instilled, edema was observed in parenchyma portion of cornea. On the other hand, as shown in Fig. 2, when the ophthalmic solution of Example 1 was instilled, little edema was observed. In the cases where the ophthalmic solutions of Example 2 or 3 are used, little edema was also observed.

These results show that the present ophthalmic compositions are effective on the oxygen deficiency of cornea and can prevent, alleviate or treat the corneal disorders caused by the oxygen deficiency.

### Test Example 2: Preservation Test of Oxygen-containing Ophthalmic Composition

Three litter of physiological saline was cooled to 4°C, bubbled with compressed oxygen for 30 minutes and filled in each container using dispenser. After filling, glass containers were closed with an aluminum screw cap furnished with polypropylene packing to yield the ophthalmic compositions. Plastic containers were closed by heating the opening under pressure to yield the ophthalmic compositions. Each composition was stored at 20°C, 60 % humidity while the change of dissolved oxygen concentration at 20°C was measured with the passage of time. The results are shown in Table 1 below. As plastic containers, those made of polyethylene terephthalate (PET), polyethylene (PE), polyethylene naphthalate (PEN) and ethylene-vinylalcohol copolymer (EVOH) were used. The dissolved oxygen amount was measured with DO meter B505 (Iijima Electronics Corporation).

**Table 1:**

| The dissolved oxygen concentration (mg/L; without oxygen replacement of void space) | | | | | | |
|---|---|---|---|---|---|---|
| Container | Day 0* | Day 1 | Day 3 | Day 7 | Day 15 | Day 30 |
| Glass | 19.20 | 19.12 | 19.08 | 19.03 | 19.11 | 19.22 |
| EVOH | 19.18 | 19.61 | 19.05 | 18.82 | 16.05 | 15.40 |
| PET | 19.61 | 19.56 | 19.03 | 18.13 | 16.13 | 15.21 |
| PEN | 19.56 | 19.26 | 19.48 | 18.95 | 16.29 | 15.87 |
| PE | 19.20 | 19.20 | 10.33 | 7.93 | 5.36 | 5.02 |

| | | | | | | |
|---|---|---|---|---|---|---|
| *Day 0: immediately after filling | | | | | | |

In the glass container, EVOH container, PET container and PEN container, the dissolved oxygen concentration decreased on days 1 and 2 following the filling, but became constant and showed almost twice as high as the saturated dissolved oxygen concentration at 20°C (8.84 mg/L) even on day 30 after the filling. On the other hand, in the PE container, the dissolved oxygen concentration at 20°C was less than the saturated dissolved oxygen concentration on day 7 after the filling.

Ophthalmic compositions were prepared and filled in containers according to a similar method as described above, and the void space in a container was replaced with oxygen. Each container was then closed tightly to give oxygen-replaced compositions.

**Table 2:**

| The dissolved oxygen concentration (mg/L; with oxygen replacement of void space) | | | | | | | | |
|---|---|---|---|---|---|---|---|---|
| Container | Day0* | Day1 | Day3 | Day7 | Day15 | Day30 | Day90 | Day300 |
| Glass | 19.32 | 20.18 | 20.03 | 19.88 | 20.13 | 20.06 | 20.03 | 20.10 |
| EVOH | 19.21 | 19.87 | 18.96 | 19.45 | 19.38 | 19.19 | 19.10 | 19.20 |
| PET | 19.33 | 20.89 | 20.17 | 19.66 | 19.07 | 19.04 | 19.02 | 19.00 |
| PEN | 19.43 | 20.14 | 20.11 | 19.87 | 19.96 | 19.16 | 19.13 | 19.12 |
| PE | 19.22 | 17.10 | 12.67 | 10.73 | 7.83 | 5.11 | - | - |

| | | | | | | | | |
|---|---|---|---|---|---|---|---|---|
| Day0*: immediately after filling | | | | | | | | |

In the glass container, EVOH container, PET container and PEN container, the dissolved oxygen concentration on day 30 was almost the same as that of at the time of filling maintaining near twice as high as the saturated dissolved oxygen concentration at 20°C (8.84 mg/L). On the other hand, in the PE container, the dissolved oxygen concentration was less than the saturated dissolved oxygen concentration at 20°C on day 15 after the filling.

### Test Example 3: Protection Effect on Corneal Imbibition

Protection effect on the corneal swelling caused by closing eyelids was examined on the basis of focusing mechanism as an index. Ten normal volunteers were examined. Five ml of test solution was taken into an eyewash cup, and each subject washed both eyes with the solution for 10 seconds and closed eyelids. After 15 minutes, both eyes were opened and compared the focusing function with that of before instillation according to the above-mentioned indexes. Following the comparison, the eyelids were closed again and, 30 minutes after the instillation, the both eyes were opened and compared the focusing function with that of before treatment according to the above-mentioned indexes. After more than 6 hours interval, the same examination was performed using the control solution.

Test solution: 3L of physiological saline was cooled to 4°C, bubbled with compressed oxygen for 30 minutes, filled in a polyethylene terephthalate container (100 ml volume) and used as a test solution. The dissolved oxygen concentration before test: 19.5 mg/L.

Control solution: physiological saline was filled in a polyethylene terephthalate container (100 ml volume) and uses as a control solution. The dissolved oxygen concentration before test: 5.4 mg/L.

Criteria for judgment are as follows:
5 = Time required for focusing is shorter and it is easier to get vision compared to the condition before instillation; 4 = time required for focusing is little shorter and it is little easier to get vision compared to the condition before instillation; 3 = there is no change in the time needed for focusing and getting vision compared to the condition before instillation; 2 = a little longer time was needed for focusing and it is a little hard to get vision compared to the condition before instillation; and 1 = longer time was needed for focusing and it is difficult to get vision compared to the condition before instillation. The results are shown in Table 3.

**Table 3**

| | 15 Minutes after instillation | 30 Minutes after instillation |
|---|---|---|
| Test solution | 2.8 | 2.5 |
| Control solution | 2.1 | 1.1 |

### Industrial Applicability

According to the present invention, it is now provided oxygen-rich ophthalmic compositions, which can be useful in prevention, alleviation or treatment of corneal disorders caused by oxygen deficiency in cornea. It becomes possible to treat diseases resulting from oxygen deficiency of cornea represented by dry eye, which diseases are related to wearing contact lens, especially soft contact lens, and use of computer, and the like.

## Claims

1. An ophthalmic composition, which is enriched with oxygen.

2. The ophthalmic composition according to claim 1, which comprises a sufficient amount of the dissolved oxygen and/or an oxygen donor to treat or prevent an ophthalmological disease resulting from oxygen deficiency.

3. The ophthalmic composition according to claim 2, wherein the amount of oxygen present as dissolved oxygen and/or that contained in oxygen donor is 10 mg or above per liter of the composition.

4. The ophthalmic composition according to claim 1 or 2, wherein the amount of oxygen present as dissolved oxygen and/or that contained in oxygen donor is 8.84 mg/1 or above per liter of the composition at 1 atmospheric pressure, 20 °C.

5. The ophthalmic composition according to claim 1 or 2, wherein the content of oxygen is at least 90% of the saturated dissolved oxygen in the presence of air at 1 atmospheric pressure.

6. The ophthalmic composition according to any one of claims 1 to 5, which is selected from the group consisting of eye drop, eyewash and contact re-wetting and/or wetting solution.

7. The ophthalmic composition according to any one of claims 2 to 6, wherein the oxygen donor is an oxygen carrier comprising captured oxygen.

8. The ophthalmic composition according to claim 7, wherein the oxygen carrier is fluorocarbon.

9. The ophthalmic composition according to claim 7, wherein the oxygen carrier is one or more substances selected from the group consisting (a) hemoglobin or a polymer thereof; (b) modified hemoglobin or a polymer thereof, (c) porphyrin complex or a polymer thereof; and (d) a lipid endoplasmic reticulum which includes hemoglobin or a polymer thereof; modified hemoglobin or a polymer thereof; or porphyrin complex or a polymer thereof;

10. The ophthalmic composition according to any one of claims 1 to 9, which further comprises at least one substance selected from the group consisting of inorganic salts, sugars, vitamins, amino acids, epithelial cell growth factors and oxygen scavengers.

11. The ophthalmic composition according to any one of claims 1 to 10, which is stored in a container impermeable or less permeable to oxygen.

12. The ophthalmic composition according to any one of claims 1 to 10, which is stored in an oxygen-permeable container that is encased in a package impermeable or less permeable to oxygen.

13. The ophthalmic composition according to claim 12, wherein the room formed between the container and the package is replaced with oxygen.

14. The ophthalmic composition according to any one of claims 11 to 13, wherein the container impermeable or less permeable to oxygen or the package impermeable or less permeable to oxygen comprises at least one substance selected from the group consisting of ethylene-vinyl alcohol copolymer, polyethylene naphthalate and polyethylene terephthalate.

15. The ophthalmic composition according to any one of claims 11 to 14, which is packaged in a unit dosage container.

16. The ophthalmic composition according to any one of claims 1 to 15, which is useful for preventing or treating keratitis superficialis punctate, corneal vascularization, corneal epithelial erosion, acute corneal epithelial edemas, corneal infiltration or corneal endotherial disorders.

17. An oxygen carrier useful for producing the ophthalmic composition according to claim 1.
